# EUROPÄISCHE PATENTSCHRIFT

(11) **EP 2 507 207 B1**
(45) Veröffentlichungstag und Bekanntmachung des Hinweises auf die Patenterteilung: **26.07.2017**
(21) Anmeldenummer: 10784510.9
(22) Anmeldetag: 26.11.2010
(51) Int. Cl.: C07C 271/12, C09D 175/04, C08G 18/81, C09D 11/101, C08K 5/205, C09D 7/12

(54) **ZUSAMMENSETZUNGEN FÜR STRAHLUNGSHÄRTENDE BESCHICHTUNGEN**
COMPOSITION FOR RADIATION-CURED COATINGS
COMPOSITION POUR DES REVÊTEMENTS SÉCHANT PAR RAYONNEMENT

(30) Priorität: 01.12.2009 DE 102009058297
(43) Veröffentlichungstag der Anmeldung: 10.10.2012
(73) Patentinhaber: Fraunhofer-Gesellschaft zur Förderung der angewandten Forschung e.V., 80686 München (DE)
(72) Erfinder: GREISIGER, Heinz, 72762 Reutlingen (DE); ENTENMANN, Marc, 70734 Fellbach (DE); SCHAUER, Thadeus, 75382 Neuhengstett (DE)
(74) Vertreter: Hoeger, Stellrecht & Partner Patentanwälte mbB
(86) Internationale Anmeldenummer: PCT/EP2010/068342
(87) Internationale Veröffentlichungsnummer: WO 2011/067183

(56) Entgegenhaltungen:
- EP-A1- 0 408 034
- EP-A1- 0 477 159
- WO-A1-2004/000794
- DE-A1- 1 916 971
- US-A1- 2006 058 431
- RUBÉN MARTÍN ET AL: "General Approach to Glycosidase Inhibitors. Enantioselective Synthesis of Deoxymannojirimycin and Swainsonine", THE JOURNAL OF ORGANIC CHEMISTRY, Bd. 70, Nr. 6, 1. März 2005 (2005-03-01), Seiten 2325-2328, XP55002515, ISSN: 0022-3263, DOI: 10.1021/jo048172s
- S. MANFREDINI ET AL: "Geiparvarin Analogs. 4.1. Synthesis and Cytostatic Activity of Geiparvarin Analogs Bearing a Carbamate Moiety or a Furocoumarin Fragment on the Alkenyl Side Chain", JOURNAL OF MEDICINAL CHEMISTRY, Bd. 37, Nr. 15, 1. Juli 1994 (1994-07-01), Seiten 2401-2405, XP55002455, ISSN: 0022-2623, DOI: 10.1021/jm00041a019
- SIMONI D ET AL: "GEIPARVARIN ANALOGUES. 2. SYNTHESIS AND CYTOSTATIC ACTIVITY OF 5-(4-ARYLBUTADIENYL)-3(2H)-FURANONES AND OF N-SUBSTITUTED 3-(4-OXO-2-FURANYL)-2-BUTEN-2-YL) CARBAMATES", JOURNAL OF MEDICINAL CHEMISTRY, AMERICAN CHEMICAL SOCIETY, US, Bd. 34, 1. Januar 1991 (1991-01-01), Seiten 3172-3176, XP002311503, ISSN: 0022-2623, DOI: DOI:10.1021/JM00115A004

## Beschreibung

Die Erfindung betrifft eine Zusammensetzung zur Verwendung in strahlungshärtenden Beschichtungen, vorzugsweise UV-radikalisch härtbaren Beschichtungszusammensetzungen.

Vernetzte Polymere werden in vielfältiger Art und Weise eingesetzt. Sie stellen wichtige Grundbestandteile vieler Werkstoffe, beispielsweise von duroplastischen Formteilen, Klebemassen, Tinten, jedoch auch von Beschichtungen aller Art dar, wobei letztlich die Art und Weise der Vernetzung und deren Dichte über mechanische und chemische Eigenschaften bzw. Stabilität entscheiden.

Strahlungshärtende Beschichtungen dienen häufig der Verbesserung der Chemikalien- und mechanischen Beständigkeit der beschichteten Substrate, insbesondere der Beständigkeit gegenüber alkalisch und sauer wirkenden Reagenzien. Hierbei sind insbesondere UV-härtende, lösemittelfreie Klarlacksysteme von Bedeutung.

Den Beschichtungen kommen insbesondere als dünne Schichten aktive, anspruchsvolle Aufgaben zu. Sie schützen darunter liegende Substrate neben den erwähnten chemischen vor allem auch vor elektrochemischen und mechanischen Einflüssen und/oder verleihen Objekten eine besondere Farbe und/oder einen besonderen Oberflächenglanz.

Insbesondere die obersten Beschichtungslagen, und hier ganz besonders die Klarlackfilme, sind einer starken Einwirkung von UV-Strahlung, Hitze, Kälte, Feuchtigkeit und Sauerstoff, als auch Säuren und Basen sowie mechanischen Belastungen ausgesetzt, weshalb hier die Erzeugung besonders stark und irreversibel vernetzter, chemisch widerstandsfähiger Schichten, welche zusätzlich hohe mechanische Beständigkeit aufweisen, wichtig ist.

Infolge der Emissionsproblematik von lösemittelbasierenden Beschichtungssystemen und dem Trend zu vereinfachten und energieeffizienten Verfahren werden strahlungshärtende Beschichtungen, insbesondere UV-härtende Systeme, unter Verwendung von sogenannten Reaktivverdünnern, welche das Bindemittel lösen und sich dann bei der Vernetzung in das Netzwerk einpolymerisieren, immer wichtiger.

Die Strahlungshärtung und insbesondere die UV-Härtung über aktive vernetzende vinylfunktionelle Gruppen findet bereits umfangreiche industrielle Anwendung. Beispielhaft seien die JP 2008-274209 A, die EP 1 333 047 A1, die JP 7-069686 A, die WO 93/09084 A1 als auch die DE 24 41 600 A1 genannt, wobei die Anzahl der weltweit erfolgenden Anmeldungen innerhalb der letzten Jahre auch die Praxisrelevanz und die wirtschaftliche Bedeutung von strahlungshärtenden Systemen generell widerspiegelt.

Eine allgemeine Abhandlung über UV-vernetzende Systeme ist in "UV-Coatings" von Reinhold Schwalm, Elsevier-Verlag, Amsterdam, 2007 gegeben.

Obwohl die mechanischen Eigenschaften, als auch der erzielbare Oberflächenglanz von strahlungshärtenden Klarlacken durchaus zufriedenstellend ist, ist die Chemikalienbeständigkeit, insbesondere die Beständigkeit gegenüber alkalischen und sauren Agenzien, insbesondere bezüglich den von der Automobilindustrie vorgegebenen Kriterien noch als unzureichend zu betrachten.

Weitere aktuelle Aspekte der UV-Härtung von Polymeren beschreibt DE 10 2006 049 764 A1, gemäß der beispielsweise wässrige Polyurethan-Dispersionen, basierend auf ungesättigten Polyestern für UV-vernetzende Systeme Verwendung finden sollen.

Die Verwendung von wässrigen Dispersionen gemäß der DE 10 2006 049 764 A1 greift zwar die Einsparung von Lösemitteln in Hinblick auf den Emissionsschutz auf, ist jedoch von Seiten der Energieeffizienz infolge des notwendigen Trockenschritts bei höheren Temperaturen oder Abtrocknungszeiten kritisch zu beurteilen.

In der DE 10 2004 053 186 A1 sind wasser- und cosolvensfreie, UV-härtende Formulierungen auf der Basis von polymerisierbaren Urethan-Acrylaten und ungesättigter Polyester beschrieben.

Polymere, basierend auf Polyestern sind jedoch aus Gründen der eingeschränkten Hydrolysestabilität gegenüber Säuren und Basen nur sehr bedingt empfehlenswert.

In der WO 2008/049932 A1 sind strahlungshärtende Mischungen beschrieben, welche als Haftklebstoffe geeignet sind und niedermolekulare ethylenische Anteile enthalten. Diese niedermolekularen Anteile werden bei der Vernetzung in das Netzwerk eingebaut. Hiermit können in der Regel sehr gut härtende Formulierungen erhalten werden, wobei es allerdings oftmals zu Unverträglichkeiten und Schwierigkeiten bezüglich des Aushärtegrades kommen kann.

In diesen Systemen werden oftmals niedermolekulare flüssige acrylat-/ methacrylatfunktionelle Verbindungen als Reaktivverdünner für die ebenfalls acrylatfunktionellen Bindemittel verwendet. Sehr gebräuchlich ist diesbezüglich insbesondere Hexandioldiacrylat (HDDA), das beispielsweise von der BASF SE unter dem Handelsnamen Laromer ® HDDA vertrieben wird.

In der WO 2004/000794 A1 werden durch die Umsetzung von ungesättigten Alkoholen mit Diisocyanaten Verbindungen mit Allylfunktionalitäten erhalten, wobei die Anbindung der Doppelbindungsfunktionalität im Molekül über jeweils ein Carbamat-Sauerstoffatom erfolgt. Der Einsatz dieser Verbindungen zeigt in der Tat eine Verbesserung der Chemikalienbeständigkeit, mit Ausnahme der Beständigkeit gegenüber Säuren, die nicht wesentlich beeinflusst wird.

Problematisch ist die Löslichkeit und Kompatibilität dieser Verbindungen in den kommerziell verfügbaren UV-vernetzenden Lacksystemen. Da diese Verbindungen über Diisocyanate synthetisiert werden, ist die Auswahl des molekularen Grundkörpers auf die kommerziell zur Verfügung stehenden Diisocyanate beschränkt, was eine gezielte Anpassung der Kompatibilität oder eine Variation der Lackeigenschaften stark limitiert.

Weiterhin nachteilig ist, dass sich multiple funktionale Anbindungen von Allylgruppen bei diesen Verbindungen schlecht in der Praxis umsetzen lassen, da zum einen die Verfügbarkeit an tri- oder höherfunktionalen Isocyanaten noch stärker als bei den Diisocyanaten begrenzt ist und zum anderen diese Verbindungen leicht zur Polymerisation neigen. Da jedoch die Anzahl von Vinyl-/Allylfunktionalitäten pro Molekül die Vernetzungsdichte und somit maßgeblich die mechanischen Eigenschaften und chemischen Resistenzen der UV-vernetzenden Lackschichten über die Funktionalität der Doppelbindungen beeinflusst, lassen sich mit diesen Verbindungen auch nur begrenzte Verbesserungen der Chemikalienbeständigkeiten erreichen.

Die WO 93/09084 A1 zielt auf Systeme für die Herstellung von Kontaktlinsen und beschreibt Vinylcarbamatgruppen-haltige Verbindungen für die UV-Härtung von Styrol-/Acrylat-/Vinylmonomermischungen für Hydrogele. Als besonders für diese Monomermischungen geeignet werden Verbindungen beschrieben, welche zumindest eine Vinylgruppe besitzen, wobei eine zweite Gruppe ausgewählt sein muss aus den Funktionalitäten einer Styrol- oder Acrylatgruppe.

Für die meisten UV-vernetzenden Lacksysteme ist jedoch eine Styrolfunktionalität kritisch zu sehen, da oftmals hieraus eine Unverträglichkeit mit bestehenden UV-vernetzenden auf HDDA basierenden Lacksystemen resultiert.

Aus der WO2004/000794 A1 sind multifunktionale Allyl-Carbamate und hieraus hergestellte Beschichtungen bekannt.

Die EP 0 477 159 A1 empfiehlt die Herstellung von Urethanen und Carbamaten ausgehend von Amin- oder Alkoholverbindungen mit Kohlendioxid und einem Allylhalogenid.

Gemäß der US 2006/0058431 A1 eignen sich sowohl Triallylisocyanurate und Triallylcyanurate als auch verschiedene Polyolpoly(meth)-acrylate als Ver-netzungsmittel in Polyamiden oder Polyestern für die Herstellung flammhem-mender Polymere.

Aufgabe der vorliegenden Erfindung ist es, eine Zusammensetzung zur Verwendung in strahlungshärtenden Beschichtungen vorzuschlagen, welche eine genügend hohe Kompatibilität mit möglichst vielen Bindemittelsystemen gewährleistet. Dabei sollte eine UV-vernetzbare Doppelbindung möglichst sterisch wenig anspruchsvoll, über flexible Bindungen angebunden und hochreaktiv gestaltet sein.

Diese Aufgabe wird von einer Zusammensetzung mit den Merkmalen des Anspruchs 1 gelöst.

Bevorzugte erfindungsgemäße Zusammensetzungen werden so konzipiert, dass diese insbesondere in acrylatbasierenden Reaktivverdünnern löslich sind.

Als besonders wichtig stellt sich heraus, dass bei der erfindungsgemäßen Zusammensetzung auch mit nicht-acrylatischen Doppelbindungsfunktionen eine gute Copolymerisation gewährleistet wird.

Die erfindungsgemäße Zusammensetzung ist sehr gut als Bestandteil von UV-vernetzenden Klarlacksystemen mit gebräuchlichen Reaktivverdünnersystemen geeignet, da sie löslich oder zumindest soweit kompatibel sind, dass Separationserscheinungen weitgehend vermieden werden können.

Weiterhin weist die erfindungsgemäße Zusammensetzung eine Doppelbindungsfunktionalität auf, welche sich mit den gebräuchlichen acrylatfunktionellen Bindemitteln und weiteren Reaktivverdünnern gut copolymerisieren lässt, wobei diese vorzugsweise zumindest teilweise über säure-/basenhydrolysestabile Bindungen angebunden sind.

In einer weiteren erfindungsgemäßen Variante ist die Allylfunktionalität angebunden an ein Stickstoffatom, welches in ein heterocyclisches System, insbesondere in eine Triazinverbindung, eingebunden ist. Die Triazinverbindungen können in tautomerer Form vorliegen. Der Begriff Triazin soll im vorliegenden Zusammenhang immer auch tautomere Formen einschließen, auch wenn im Folgenden der Einfachheit halber nicht immer ein expliziter Hinweis darauf erfolgt.

Das beschriebene N-allylfunktionale Triazin der Formel 1 lässt sich unter entsprechender basischer Katalyse aus Allylisocyanaten einfach herstellen und ist kommerziell erhältlich.

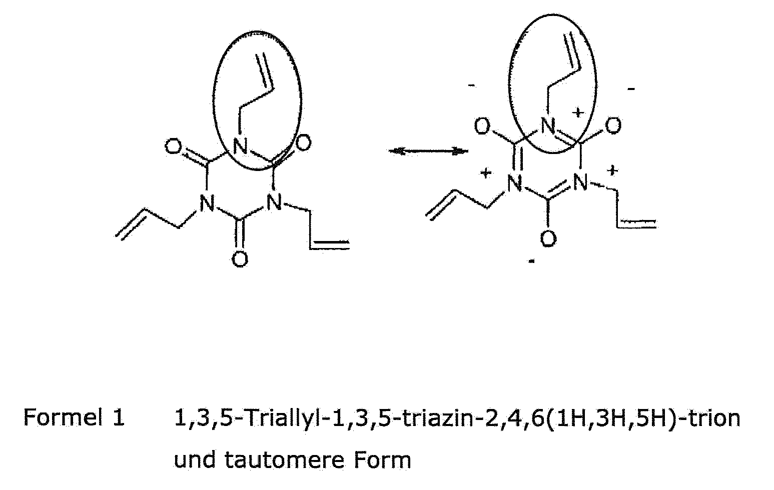

Besonders bevorzugt ist der Einsatz dieser Verbindungen in lösemittelfreien UV-härtenden Klarlackformulierungen (siehe Beispiel 6), da hier infolge der hohen Anforderungen an Transparenz andere Additive, beispielsweise puffernde oder matrixverstärkende Pigmente und Füllstoffe, nicht zum Einsatz kommen können.

Die N-allylfunktionalen Verbindungen finden auch Verwendung in UV-vernetzenden Tinten, Pulverlacken oder generell in UV-vernetzenden Polymermatrizen aller Art.

Die erfindungsgemäßen Zusammensetzungen sind lösemittelfrei.

Der Einsatz von Triallylisocyanat wird herkömmlich in Verbindung mit organischen Schwefelverbindungen in UV-härtenden Systemen beschrieben (siehe z.B. JP 57-133108 A, JP 57-158230 A, JP 58-213022 A, FR 2258436 A1, DE 24 02 390 A1, US 3,855,093 A), wobei der Härteprozess nicht wie bei der vorliegenden Erfindung UV-radikalisch, sondern UV-kationisch verläuft.

Die Verwendung der zumeist giftigen und ökologisch bedenklichen organischen Schwefelverbindungen muss hierbei jedoch zukünftig kritisch gesehen werden, da gerade die Beschichtungsindustrie an Konzepten der Emissionsfreiheit, als auch zur Abwasserreinhaltung interessiert ist. Der Einsatz von UV-härtenden Klarlackschichten, welche jedoch organische Schwefelverbindungen beinhalten, widerspricht einem solchen Konzept und ist für eine umweltfreundliche Beschichtungstechnologie weniger geeignet, zumal sich die Schwefelverbindungen zum Teil aus der Beschichtung herauslösen können.

Die in den vorgenannten Druckschriften beschriebenen Verfahren zur UV-Härtung von Polymeren stellen für strahlungshärtende Lackschichten, insbesondere für die ökologisch und ökonomisch interessanten UV-härtenden Klarlacke, somit keine sinnvolle Alternativlösung dar.

Triallylisocyanat wurde ferner als Bestandteil von UV-radikalisch härtenden Mischungen zur Herstellung von Thermoplasten und/oder Elastomeren in EP 1 111 008 A1, EP 1 338 623 A1 und EP 1 674 513 A1 beschrieben.

Diese Verfahren betreffen im wesentlichen dickschichtige, unvernetzte oder schwach vernetzte Polymeranwendungen und sind ungeeignet zur Herstellung von dünnschichtigen, stark vernetzten, gegenüber UV- und Wettereinflüssen stabilisierten, als auch mechanisch beständigen Beschichtungen.

Um die Anforderungen an die resultierenden Beschichtungen und Klarlackschichten zu erfüllen, sind Verbindungen mit höherem Molekulargewicht weniger geeignet, da generell die Löslichkeit und Kompatibilität mit zunehmender Molmasse in den etablierten strahlungshärtenden Formulierungen in der Regel abnimmt, während die Viskosität stark ansteigt.

Ein starker Viskositätsanstieg in den UV-Lackpräparationen ist zumeist verbunden mit einem schlechten Verlauf, niedrigen Oberflächenglanzwerten und auch geringeren mechanischen und chemischen Stabilitäten, was für Klarlackbeschichtungen besonders problematisch ist.

Bevorzugt werden deshalb niedermolekulare und insbesondere monomolekulare Verbindungen mit einem Molekulargewicht unter 5000 g/mol, weiterhin bevorzugt unter 1000 g/mol und insbesondere mit einem Molekulargewicht unter 700 g/mol verwendet.

Auch Mischungen der beschriebenen N-allylfunktionalen Stoffe können in speziellen Zusammensetzungen vorteilhaft sein, um die Einpolymerisation der Einzelkomponenten durch Copolymerisation stark zu verbessern.

Aus der vorstehenden Erläuterung der erfindungsgemäßen Hexamethylendioldiacrylatzusammensetzungen wurde bereits deutlich, dass ein weiterer wesentlicher Aspekt der vorliegenden Erfindung in der Bereitstellung von Beschichtungszusammensetzungen besteht, wie sie in Anspruch 3 definiert sind.

Bevorzugt sind demnach im Wesentlichen lösemittelfreie Zusammensetzungen. Diese Zusammensetzungen können insbesondere auch einen oder mehrere weitere Reaktivverdünner umfassen.

Im Rahmen der Erfindung geeignete Reaktivverdünner sind Hexamethylendioldiacrylat (HDDA), Hexamethylendioldimethacrylat (HDDMA), Isobornylacrylat (IBOA), Tripropylenglykoldiacrylat (TPGDA) und Trimethylolpropantriacrylat (TMPTA).

In der Mischung der erfindungsgemäßen Zusammensetzung mit einem weiteren Reaktivverdünner beträgt der Anteil der N-allylfunktionalen 1,3,5-Triazin-Verbindungen vorzugsweise ca. 5 bis ca. 80 Gew.% sowie bei manchen Applikationen auch mehr.

Des Weiteren umfassen die erfindungsgemäßen Beschichtungszusammensetzungen bevorzugt ein polymer- oder oligomer-basierendes Bindemittel.

Solche Bindemittel umfassen bevorzugt eine acrylat-, urethan- oder polyesterfunktionelle Bindemittelkomponente.

Die erfindungsgemäßen Beschichtungszusammensetzungen eignen sich insbesondere für die Herstellung von Beschichtungen mit Schichtdicken von ca. 0,5 µm bis ca. 600 µm, weiter bevorzugt ca. 0,5 µm bis ca.100 µm.

Die erfindungsgemäßen Beschichtungszusammensetzungen sind weiter bevorzugt als Lack, insbesondere als Klarlack, formuliert. Hierbei sind besonders bevorzugt Klarlackformulierungen, die UV-härtbar, noch weiter bevorzugt UV-radikalisch härtbar sind.

Die erfindungsgemäßen Beschichtungszusammensetzungen sind ebenfalls bevorzugt als Tinte zu formulieren.

Die vorgenannten erfindungsgemäßen Beschichtungszusammensetzungen zeichnen sich bevorzugt dadurch aus, dass der Anteil der 1,3,5-Triazin-Verbindung ca. 80 Gew.-% oder weniger, insbesondere ca. 5 bis 60 Gew.-%, weiter bevorzugt ca. 25 bis 60 Gew.-%, am meisten bevorzugt ca. 30 bis 60 Gew.% beträgt.

Folgende Beispiele sollen die Erfindung noch näher erläutern, ohne sie jedoch zu beschränken:

### Beispiele:

### Referenz-Beispiel 1: Synthese von Hexamethylen-bis-N-allylcarbamat

11,4 g Hexamethylendiol und 0,01216 g Dibuthylzinndodecanat (Katalysator) werden in 70 g Methylenchlorid gelöst, und es werden unter Eis-Kühlung 16,0 g Allylisocyanat zugetropft.

Nach 20 Stunden Rühren unter Stickstoffatmosphäre bei Raumtemperatur wird das Methylenchlorid im Vakuum abgezogen. Das erhaltene Produkt wird mit 50 °C warmem destilliertem Wasser gewaschen, bis das Waschwasser pH-neutral ist.

Danach wird mit NaSO₄ getrocknet. Die Produktidentifizierung erfolgte durch Protonenresonanzspektroskopie.

### Referenz-Beispiel 2: Synthese von Acryloyl-hexamethylen-N-allylcarbamat

12,8 g Hexamethylendiol werden in 150 g Methylenchlorid gelöst, und es werden unter Eis-Kühlung 9,0 g Allylisocyanat zugetropft.

Nach 20 Stunden Erwärmen auf 35 °C unter Stickstoffatmosphäre wird wiederholt mit Eis-Wasser gekühlt, 15 ml Di-isopropylethylamin zugegeben und 9,8 g Acryloylchlorid zugetropft.

Es wird 20 Stunden bei Raumtemperatur gerührt und das Methylenchlorid im Vakuum abgezogen.

Das entstehende Produkt wird durch Schütteln mit kaltem Wasser gewaschen, bis das Waschwasser eine neutrale pH-Reaktion zeigt.

Danach wird mit ungefähr der gleichen Menge Butylacetat verdünnt und noch dreimal mit kaltem Wasser ausgeschüttelt.

Nach dem Trocknen über Magnesiumchlorid wird das Butylacetat vorsichtig im Vakuum bei möglichst geringer Temperatur abgezogen. Die Produktidentifizierung erfolgte durch Protonenresonanzspektroskopie.

### Referenz-Beispiel 3: Synthese von 1,4-Tetramethylen-bis-(di-allylamino)-carbamat

Zu einer mit Eiswasser gekühlten Lösung von 10,0 g Butandiol-bis-chloroformiat in 54 ml Methylenchlorid wird eine Lösung von 10,0 g Di-allylamin und nachfolgend 12 ml Di-isopropylethylamin in 80 ml Methylenchlorid zutropft.

Nach 20 Stunden Rühren bei Raumtemperatur wird unter Vakuum das Methylenchlorid abgezogen und vorsichtig mit 50 °C warmem destilliertem Wasser solange gewaschen, bis das Waschwasser pH-neutral ist.

Danach wird mit NaSO₄ getrocknet. Die Produktidentifizierung erfolgte durch Protonenresonanzspektroskopie.

### Referenz-Beispiel 4: Synthese von 1,6-Hexamethylen-bis-allylcarbamat (gemäß WO 2004/000794 A1)

Zu 14,0 g Allylalkohol und 0,0152 g Dibuthylzinndodecanat in 70 g Methylenchlorid werden unter Eis-Kühlung 20,0 g Hexamethylendiisocyanat zugetropft.

Nach 20 Stunden Rühren unter Stickstoffatmosphäre bei Raumtemperatur wird, nach Abziehen des Methylenchlorid unter Vakuum, durch mehrmaliges Schütteln mit 50 °C warmem destilliertem Wasser solange gewaschen, bis das Waschwasser pH-neutral ist.

Danach wird mit NaSO₄ getrocknet. Die Produktidentifizierung erfolgte durch Protonenresonanzspektroskopie.

### Referenz-Beispiel 5: Synthese von N,N-Isophoron-bis-allylcarbamat (gemäß WO 2004/000794 A1)

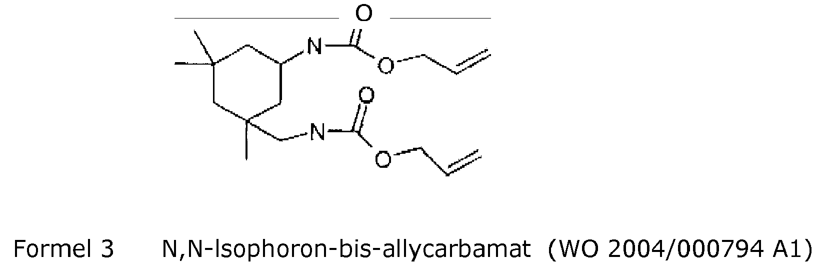

Zu 14,0 g Allylalkohol und 0,0152 g Dibuthylzinndodecanat werden unter Eis-Kühlung 26,7 g Isophoron-diisocyanat zugetropft.

Nach 20 Stunden Rühren unter Stickstoffatmosphäre bei Raumtemperatur wird durch mehrmaliges Schütteln mit 50 °C warmem, destilliertem Wasser solange gewaschen, bis das Waschwasser pH-neutral ist.

Danach wird mit NaSO₄ getrocknet. Die Produktidentifizierung erfolgte durch Protonenresonanzspektroskopie.

### Beispiel 6: Verarbeitung der Verbindungen aus den Referenz-Beispielen 1 bis 5 und zusätzlicher kommerziell erhältlicher Komponenten zu Beschichtungsformulierungen

Als Bindemittel für die strahlungshärtenden Testformulierungen dieses Beispiels wurde Desmolux® U 880H von Bayer MaterialScience, als Reaktivverdünner Laromer® HDDA von BASF und als Verlaufsadditiv BYK 306 von BYK Altana verwendet.

Zur Herstellung der Beschichtungsformulierungen wurden die oben genannten Beschichtungskomponenten, der UV-Vernetzer Irgacure 184 von Ciba, sowie gegebenenfalls eine in den Referenz-Beispielen 1 bis 5 erhaltene Verbindung bzw. 1,3,5-Triallyl-1,3,5-triazine-2,4,6(1H,3H,5H)-trion (Aldrich) als N-allylfunktionale Triazinverbindung oder 2,4,6-Triallyloxy-1,3,5-triazin (Aldrich) als O-allylfunktionale Triazinverbindung am Hochgeschwindigkeitsrührer mit einer Zahnscheibe für die Dauer von 15 min bei einer Umfangsgeschwindigkeit der Scheibe von 1,0 m/sec, entsprechend den in Tabelle 1 angegebenen Anteilen, gemischt.

Die in Tabelle 1 angegebene Standardformulierung stellt die Grundformulierung dar, welche jeweils durch einen Austausch von 50 % der HDDA-Komponente variiert wurden, um die Verbindungen der Referenz-Beispiele 1 bis 5 und der weiteren beiden Triazinverbindungen bezüglich ihrer Wirkungsweise auf das Beschichtungssystem zu testen, die in der Tabelle als Austausch-Additive bezeichnet sind.

Appliziert wurden die erhaltenen Testformulierungen unter Verwendung eines 50 µm Spaltrakels auf schwarz vorlackierte Bleche (z.B. schwarzer Anteil von unter der Bezeichnung Leneta-Blech Form M12 black and whight spray monitor erhältlichen Blechen).

Die Strahlungshärtung erfolgte durch UV-Bestrahlung (undotierte Hg-Hochdrucklampe) der applizierten Lackschichten bei Raumtemperatur mit einer UV-Dosis von 1800 mJ/cm² in einer inerten N₂-Atmosphäre.

**Tabelle 1**

| Lackkomponenten | Standard-Rezeptur | modifizierte Rezeptur |
|---|---|---|
| Desmolux U 880H [Gew.%] | 59,70 | 59,70 |
| Byk 306 [Gew.%] | 0,93 | 0,93 |
| Irgacure 184 [Gew.%] | 2,89 | 2,89 |
| Laromer HDDA [Gew.%] | 36,48 | 18,24 |
| Austausch-Additiv [Gew.%] | - | 18,24 |

### Beispiel 7: Ergebnisse der Prüfungen der Lackschichten

Die Abtestung der Alkali-/Säurenbeständigkeit der in Beispiel 6 hergestellten Lackproben erfolgte durch Auftropfen einer einprozentigen Natronlauge bzw. Schwefelsäurelösung.

Die so behandelten Beschichtungen wurden in einem Gradientenofen für 30 min einem Temperaturgradienten ausgesetzt. Nach dem Spülen der belasteten Flächen mit Wasser und Trocknung erfolgte nach 24 Stunden Lagerung bei 23 °C bei 50 % rel. Luftfeuchte eine visuelle Bewertung der hervorgerufenen Schädigung.

Es wurde für die Alkali- und Säurebeständigkeit der Beschichtung die niedrigste Temperatur bestimmt, für welche visuell eine Schädigung erkennbar war.

Vergleicht man nun die erhaltenen Säure- und Alkalistabilitäten der Standardformulierung mit den Stabilitäten für die Beschichtungen, welche durch Austausch von 50 % des HDDA-Anteils durch die Austausch-Additive entstehen, so erhält man für die Standardformulierung mit HDDA eine alkalische Temperaturstabilität von 41 °C und eine Säurestabilität von 42 °C.

Für die mit der N-allylfunktionellen Triazinverbindung modifizierte Lackschicht wird eine hohe alkalische Temperaturstabilität von 61 °C und eine erhöhte Säurestabilität von 47 °C detektiert.

Für die O-allylfunktionelle Triazinverbindung wird bei 50 % Austausch mit HDDA eine gegenüber dem N-allylfunktionalen Triazin erniedrigte alkalische Temperaturstabilität von 59 °C und eine ebenfalls erniedrigte Säurebeständigkeit von 44 °C als Ergebnis erhalten.

Jedoch nicht nur bei den Chemikalienbeständigkeiten zeigt die N-allylfunktionale Triazinverbindung gegenüber der O-allylfunktionellen Triazinverbindung Vorteile. Wird eine 300 h Kurzzeitbewitterung in Anlehnung an DIN EN ISO 11341 der erhaltenen Lackschichten durchgeführt, so lässt sich bei Einsatz des O-allylfunktionellen Triazins eine um 0,35 Einheiten stärkere Farbänderung ΔE nach der Belastung beobachten (Messgeometrie d/8°, color I5, GretagMacbeth).

Dies spricht für eine höhere Bewitterungsstabilität von N-allylfunktionalen Triazinen gegenüber O-allylfunktionalen Triazin-Verbindungen.

Werden weitere O-allylfunktionale Stoffe, welche sich gemäß der zitierten WO 2004/000794 A1 aus Diisocyanaten und Allylalkoholen herstellen lassen, für die Eignung in einer UV-härtenden Klarlackanwendung untersucht, so wird beim Einsatz des cyclischen N,N-Isophoron-bis-allylcarbamat aus Beispiel 5 (Formel 3) bei einem 50 % Austausch von HDDA nur eine alkalische Temperaturstabilität von 54 °C und eine Säurestabilität von ebenfalls nur 44 °C erreicht.

Analog zu dem N,N-Isophoron-bis-allylcarbamat wird durch Umsetzung des entsprechenden Hexamethylendiisocyanats mit Allylalkohol gemäß WO 2004/000794 A1 das Hexamethylen-bis-allylcarbamat (Referenz-Beispiel 4) erhalten. Diese Probe ist inkompatibel mit dem Lacksystem und lässt sich nicht direkt in HDDA lösen, wobei das entsprechende N-allylfunktionelle Hexamethylen-bis-N-allylcarbamat aus Beispiel 1 sich ebenfalls nicht direkt in HDDA lösen lässt, so dass insgesamt auf eine inkompatibilisierende Wirkung des Hexamethylen-Grundkörpers geschlossen werden kann.

Um dennoch eine Abtestung des Einflusses auf die Chemikafienbeständigkeit vornehmen zu können wurde das Hexamethylen-bis-allylcarbamat aus Referenz-Beispiel 4 zuvor in Methanol gelöst, appliziert und die Lackschicht vor der UV-Vernetzung im Vakuum bei 50 °C zur Entfernung des Methanols für mehrere Stunden getrocknet.

Es wurden zwar transparente Lackschichten erhalten, überraschenderweise wurde jedoch nur eine gegenüber dem Standardlack leicht verbesserte Alkalibeständigkeit von 45 °C und eine Säurebeständigkeit von 44 °C erreicht.

Wird nun ein Teil der Allyl-Carbamatfunktionen erfindungsgemäß durch Acrylatgruppen ausgetauscht, werden die Verbindungen kompatibler mit dem Lacksystem und die Alkalibeständigkeit der Lackschicht steigt bei Einarbeitung des aus Referenz-Beispiel 2 erhaltenen Stoffes gegenüber dem gemäß WO 2004/000794 A1 eingesetzten Hexamethylen-bis-allylcarbamat aus Beispiel 4 überraschenderweise von 45 °C auf 48 °C an, während die Säurebeständigkeit mit 44 °C konstant bleibt.

Aufgrund dieses Ergebnisses kann geschlossen werden, dass die Kompatibilität einer Verbindung offenbar stark die erzielbare Chemikalienbeständigkeit der Lackschicht beeinflusst und dass der Einfluss der Kompatibilität einer Verbindung sich stärker auswirken kann, als eine hydrolysestabile Anbindung der Doppelbindungsfunktionalität.

Weiterhin erscheint eine Vereinigung gut copolymerisierender Monomereinheiten im gleichen Molekül sich positiv auf die erreichbare Chemikalienbeständigkeit auszuwirken.

Um den Einfluss des Hexamethylengrundkörpers und der Doppelbindungsfunktionalität eingehender abzuklären wurde in Referenz-Beispiel 3 über ein Tetramethylendiolchloroformat ein entsprechendes tetrafunktionelles N-Allylcarbamat, das 1,4-Tetramethylen-bis-(di-allylamino)-carbamat synthetisiert. Die erhaltene Verbindung war bei Raumtemperatur flüssig und vollkommen mischbar mit dem Reaktivverdünner HDDA. Für das resultierende Lacksystem wurde für die Alkalibeständigkeit ein relativ hoher Wert von 56 °C erhalten, während für die Säurebeständigkeit weiterhin 44 °C bestimmt wurden.

Es ist auch möglich das aus Referenz-Beispiel 3 erhaltene tetrafunktionale N-Allylcarbamat ohne HDDA direkt als alleinigen Reaktivverdünner einzusetzen, da es als Flüssigkeit vorliegt und ein gutes Lösevermögen für UV-Initiatoren und eine hohe Kompatibilität mit gängigen Bindemittelsystemen zeigt. Dies erlaubt auch den Einsatz in hohen Konzentrationen, beispielsweise mit 80 Gew.%-Anteil, in der Beschichtungszusammensetzung.

Fasst man die erhaltenen Ergebnisse zusammen, so lässt sich feststellen, dass sowohl die hydrolysestabile Anknüpfung der zu vernetzenden Doppelbindungsfunktionen, deren Funktionalität im Molekül, als auch der verwendete Grundkörper eine wichtige Rolle spielen.

Die Ergebnisse deuten an, dass die Verwendung von Ringstrukturen, insbesondere von Heterocyclen und weiterhin bevorzugt von Triazin-Verbindungen als Grundkörper, funktionalisiert mit höherfunktionalen, hydrolysestabil über Carbamatgruppen angeknüpften Allylgruppen, sich besonders vorteilhaft für eine sehr hohe Säure- und Basenstabilität auswirkt.

Um jedoch eine ausgezeichnete Chemikalienstabilität zu erreichen, kann erfindungsgemäß zusätzlich die erwähnte Kompatibilität und Reaktivität einer entsprechenden Verbindung, beispielsweise durch zusätzliche Modifizierung mit Acrylatfunktionalitäten, gezielt an das vorliegende Lacksystem angepasst werden.

Hierbei ist auf das richtige Verhältnis von hydrolysestabilen Allylcarbamatfunktionalitäten zu den Acrylatfunktionalitäten im Lacksystem zu achten, damit sich eine optimale Copolymerisation der Komponenten erreichen lässt.

Sollen die Additive Anwendung in bestehenden Lackformulierungen finden, so sollten andere Lackeigenschaften nicht negativ durch den Additivzusatz beeinflusst werden.

Überraschenderweise wurde für die mit dem N-allylfunktionalen Triazin hergestellte Lackschicht zusätzlich auch eine verbesserte Kratzfestigkeit von fast 53 mN bis zur ersten Rissbildung (Rissbildung Standardbeschichtung bei 51 mN), gemessen mit einem Gerät des Typs UNHT von CSM Instruments.

Für die mit dem O-allylfunktionalen Triazin modifizierte Lackschicht wurde eine extrem verringerte Kratzfestigkeit bei einer ersten Rissbildung schon bei 31 mN beobachtet.

Die oben beschriebenen Testergebnisse sind in der Tabelle 2 zusammengefasst.

**Tabelle 2**

| Lackzusammensetzung mit Austausch-Additiv | Laugenbeständigkeit [°C] | Säurenbeständigkeit [°C] | Kratzfestigkeit [mN] |
|---|---|---|---|
| ohne (Standard) | 41 | 42 | 51 |
| von Beispiel 1 (Referenz) | inkompatibel mit Lacksystem | | |
| von Beispiel 2 (Referenz) | 48 | 44 | - **) |
| von Beispiel 3 (Referenz) | 56 | 44 | - **) |
| von Beispiel 4*) (Referenz) | 45 | 44 | -**) |
| von Beispiel 5 (Referenz) | 54 | 44 | 50 |
| der Formel 1 N-allylfunktionales Triazin | 61 | 47 | ∼53 |
| der Formel 2 (Referenz) O-allylfunktionales Triazin | 59 | 44 | 31 |

| | | | |
|---|---|---|---|
| *) modifizierte Verarbeitung, siehe Beschreibung **) Werte wurden nicht bestimmt | | | |

Bei der Abtestung der wichtigen Lackeigenschaften, wie z.B. Oberflächenglanz und Glanzverlust (HazeGloss, Byk Gardner) oder Härte und Härteverlust nach Bewitterung (Fischerscope H100, Fischer), konnten für die erfindungsgemäßen Additive bis zu einem 50 % Austausch von HDDA in der getesteten Formulierung keine negativen Einflüsse auf die Lackschicht festgestellt werden.

Die neuartigen Additive verbessern deshalb vor allem die Chemikalienbeständigkeit und die mechanischen Beständigkeiten, ohne jedoch die Bewitterungsstabilitäten oder den Oberflächenglanz wesentlich zu beeinflussen. Sie sind infolge dessen als Zusätze in Lacksystemen, insbesondere in UV-Klarlacksystemen besonders vorteilhaft zu verwenden.

## Patentansprüche

1. Zusammensetzung zur Verwendung in strahlungshärtenden Beschichtungen, umfassend
eine 1,3,5-Triazin-Verbindung, ausgewählt aus 1,3,5-Triallyl-1,3,5-triazin-2,4,6(1H,3H,5H)-trion und dessen tautomerer Form, und einen Reaktivverdünner, ausgewählt aus Hexamethylendioldiacrylat (HDDA), Hexamethylendioldimethacrylat (HDDMA), Isobornylacrylat (IBOA), Tripropylenglykoldiacrylat (TPGDA) und Trimethylolpropantriacrylat (TMPTA), wobei die Zusammensetzung lösemittelfrei ist.

2. Zusammensetzung nach Anspruch 1, wobei die Zusammensetzung aus Verbindungen mit einer Molmasse von 5000 g/mol oder weniger, weiter bevorzugt 1000 g/mol oder weniger besteht.

3. Beschichtungszusammensetzung umfassend eine Zusammensetzung nach Anspruch 1 oder 2.

4. Beschichtungszusammensetzung nach Anspruch 3, wobei sie lösemittelfrei ist.

5. Beschichtungszusammensetzung nach Anspruch 3 oder 4, wobei sie ein polymer- oder oligomer-basierendes Bindemittel umfasst.

6. Beschichtungszusammensetzung nach Anspruch 5, wobei das Bindemittel eine acrylat-, urethan- oder polyesterfunktionelle Bindemittelkomponente umfasst.

7. Beschichtungszusammensetzung nach einem der Ansprüche 3 bis 6, wobei die Beschichtungszusammensetzung als Lack, insbesondere als Klarlack, weiter bevorzugt als UV-härtbarer Klarlack formuliert ist.

8. Beschichtungszusammensetzung nach einem der Ansprüche 3 bis 7, wobei sie UV-radikalisch härtbar ist.

9. Beschichtungszusammensetzung nach einem der Ansprüche 3 bis 8, wobei sie als Tinte formuliert ist.

10. Beschichtungszusammensetzung nach einem der Ansprüche 3 bis 9, wobei der Anteil der 1,3,5-Triazin-Verbindung 80 Gew.-% oder weniger, insbesondere 5 bis 60 Gew.-%, weiter bevorzugt 25 bis 60 Gew.-%, beträgt.

11. Verwendung einer Beschichtungszusammensetzung nach Anspruch 10 für die Herstellung von Beschichtungen mit einer Schichtdicke von 0,5 µm bis 600 µm.

## Claims

1. Composition for use in radiation-curing coatings, comprising
a 1,3,5-triazine compound selected from 1,3,5-triallyl-1,3,5-triazine-2,4,6(1H,3H,5H)trione and the tautomeric form thereof and a reactive diluent selected from hexamethylene diol diacrylate (HDDA), hexamethylene diol dimethacrylate (HDDMA), isobornyl acrylate (IBOA), tripropylene glycol diacrylate (TPGDA), and trimethylolpropane triacrylate (TMPTA), wherein the composition is solvent-free.

2. Composition according to claim 1, wherein the composition is composed of compounds having a molar mass of 5000 g/mol or less, more preferably 1000 g/mol or less.

3. Coating composition comprising a composition according to claim 1 or 2.

4. Coating composition according to claim 3, wherein the coating composition is solvent-free.

5. Coating composition according to claim 3 or 4, wherein the coating composition includes a polymer- or oligomer-based binder.

6. Coating composition according to claim 5, wherein the binder includes an acrylate-, urethane-, or polyester-functional binder component.

7. Coating composition according to any one of claims 3 to 6, wherein the coating composition is formulated as paint, in particular as a clear coat, more preferably as a UV-curable clear coat.

8. Coating composition according to any one of claims 3 to 7, wherein the coating composition is UV radical-curable.

9. Coating composition according to any one of claims 3 to 8, wherein the coating composition is formulated as ink.

10. Coating composition according to any one of claims 3 to 9, wherein the proportion of the 1,3,5-triazine compound is 80% by weight or less, in particular 5 to 60% by weight, more preferably 25 to 60% by weight.

11. Use of a coating composition according to claim 10 for the production of coatings having a film thickness in the range from 0.5 µm to 600 µm.

## Revendications

1. Composition pour l'utilisation dans des revêtements durcissables par rayonnement, comprenant un composé 1,3,5-triazine, choisi parmi la 1,3,5-triallyl-1,3,5-triazine-2,4,6(1H,3H,5H)-trione et sa forme tautomère, et un diluant réactif, choisi parmi le diacrylate d'hexaméthylènediol (HDDA), le diméthacrylate d'hexaméthylènediol (HDDMA), l'acrylate d'isobornyle (IBOA), le diacrylate de tripropylène glycol (TPGDA) et le triacrylate de triméthylolpropane (TMPTA), la composition étant dépourvue de solvants.

2. Composition selon la revendication 1, qui est constituée de composés ayant une masse molaire de 5 000 g/mole ou moins, plus préférentiellement de 1 000 g/mole ou moins.

3. Composition de revêtement comprenant une composition selon la revendication 1 ou 2.

4. Composition de revêtement selon la revendication 3, qui est dépourvue de solvants.

5. Composition de revêtement selon la revendication 3 ou 4, qui comprend un liant à base de polymère ou d'oligomère.

6. Composition de revêtement selon la revendication 5, dans laquelle le liant comprend un composant de liant ayant une fonction acrylate, uréthane ou polyester.

7. Composition de revêtement selon l'une des revendications 3 à 6, qui est formulée sous forme de laque, en particulier de vernis, plus préférentiellement de vernis durcissable par UV.

8. Composition de revêtement selon l'une des revendications 3 à 7, qui est durcissable par voie radicalaire - UV.

9. Composition de revêtement selon l'une des revendications 3 à 8, qui est formulée sous forme d'encre.

10. Composition de revêtement selon l'une des revendications 3 à 9, dans laquelle la proportion de composé 1,3,5-triazine est de 80 % en poids ou moins, en particulier de 5 à 60 % en poids, plus préférentiellement de 25 à 60 % en poids.

11. Utilisation d'une composition de revêtement selon la revendication 10 pour la fabrication de revêtements ayant une épaisseur de couche de 0,5 µm à 600 µm.
